# EUROPEAN PATENT SPECIFICATION

(11) **EP 1 721 532 B1**
(45) Date of publication and mention of the grant of the patent: **03.06.2009**
(21) Application number: 06003982.3
(22) Date of filing: 27.02.2006
(51) Int. Cl.: A23L 1/38, A61K 36/00

(54) **Ellagic acid food supplement prepared from pomegranate seed**
Nahrungsergänzungsmittel enthaltend Ellagsäure aus Granatapfel
Supplément alimentaire dérivé de la graine de grenade et contenant de l'acide ellagique

(30) Priority: 09.05.2005 US 125596
(43) Date of publication of application: 15.11.2006
(73) Proprietor: Collagen Nutraceuticals, Inc., Lake Forest, CA 92630 (US)
(72) Inventor: Alkayali, Ahmad, Lake Forest, CA 92630 (US)
(74) Representative: Winter, Brandl, Fürniss, Hübner Röss, Kaiser, Polte Partnerschaft Patent- und Rechtsanwaltskanzlei

(56) References cited:
- US-A1- 2002 197 341
- LANSKY EPHRAIM PHILIP ET AL: "Pomegranate (Punica granatum) pure chemicals show possible synergistic inhibition of human PC-3 prostate cancer cell invasion across Matrigel." INVESTIGATIONAL NEW DRUGS. MAR 2005, vol. 23, no. 2, March 2005 (2005-03), pages 121-122, XP002389040 ISSN: 0167-6997
- YOSHIMURA MINEKA ET AL: "Inhibitory effect of an ellagic acid-rich pomegranate extract on tyrosinase activity and ultraviolet-induced pigmentation" BIOSCIENCE BIOTECHNOLOGY AND BIOCHEMISTRY, vol. 69, no. 12, December 2005 (2005-12), pages 2368-2373, XP002389041 ISSN: 0916-8451
- MALIK ARSHI ET AL: "Pomegranate fruit juice for chemoprevention and chemotherapy of prostate cancer" PROCEEDINGS OF THE NATIONAL ACADEMY OF SCIENCES OF THE UNITED STATES OF AMERICA, vol. 102, no. 41, October 2005 (2005-10), pages 14813-14818, XP002389042 ISSN: 0027-8424
- LI ET AL: "Evaluation of antioxidant properties of pomegranate peel extract in comparison with pomegranate pulp extract" FOOD CHEMISTRY, ELSEVIER SCIENCE PUBLISHERS LTD, GB, vol. 96, no. 2, May 2006 (2006-05), pages 254-260, XP005137065 ISSN: 0308-8146
- ASLAM ET AL: "Pomegranate as a cosmeceutical source: Pomegranate fractions promote proliferation and procollagen synthesis and inhibit matrix metalloproteinase-1 production in human skin cells" JOURNAL OF ETHNOPHARMACOLOGY, ELSEVIER SCIENTIFIC PUBLISHERS LTD, IE, vol. 103, no. 3, 20 February 2006 (2006-02-20), pages 311-318, XP005263298 ISSN: 0378-8741
- LIANG CHIA-PEI ET AL: "Antioxidant activity of plant extracts on the inhibition of citral off-odor formation." MOLECULAR NUTRITION & FOOD RESEARCH. SEP 2004, vol. 48, no. 4, September 2004 (2004-09), pages 308-317, XP002389043 ISSN: 1613-4125

## Description

### FIELD OF THE INVENTION

The present invention relates to a method of preparing ellagic acid (in the form of pomegranate polyphenol extract) from pomegranate seed. The product material is preferably prepared in the form of a fine powder. And, the invention further relates to the beneficial use of the product material as a human nutritional or food supplement.

### BACKGROUND OF THE INVENTION

Ellagic acid is a naturally-occurring phytochemical pesticide found in a variety plant species. This biochemical serves a number of possible functions in these plants, such as regulating plant growth and seed germination, and protecting the plant from microbial infections. Ellagic acid may also protect plants from cancer-inducing chemicals, heavy metal poisoning, and predation by insects and insect larvae. Since the early 1990s, researchers have been discovering that ellagic acid offers health benefits to humans as well.

Early evidence shows that ellagic acid acts as a scavenger to "bind" or chemically engage cancer-causing chemicals, making them inactive. In addition, ellagic acid is a fused bi-nuclear coumarin derivative, which appears to prevent the binding of carcinogens to DNA, and also appears to reduce the incidence of cancer in cultured human cells exposed to carcinogens.

Although ellagic acid is the bioactive agent that offers protection, the phytochemical is generally ingested in the form of another biochemical called ellagitannin. Plants produce ellagic acid and glucose that combine to form ellagitannins, which are water-soluble compounds that are bio-available, or easier for animals to absorb in their diets.

For example, the Hollings Cancer Institute at the University of South Carolina conducted a double blind study on a large group of 500 cervical cancer patients. Nine years of study have shown that ellagic acid causes G-arrest within 48 hours (inhibiting and stopping mitosis-cancer cell division); and leads to apoptosis (normal cell death) within 72 hours, for breast, pancreas, esophageal, skin, colon and prostate cancer cells. Clinical tests also showed that ellagic acid prevents the destruction of the p53 gene by cancer cells. Additional studies suggest that one of the mechanisms by which ellagic acid inhibits mutagenesis and carcinogenesis is by forming adducts with DNA, thus masking binding sites to be occupied by the mutagen or carcinogen.

Further a publication entitled, "The American Cancer Society's Guide to Complementary and Alternative Cancer Methods" has documented that ellagic acid is a very promising natural supplement, because it causes apoptosis (cell death) of cancer cells in the lab tests, with no change to normal healthy cells. Moreover, is appears that healthy cells have a normal life cycle of approximately 120 days before they die. This process is called apoptosis (natural cell death). The body replaces these dying cells with healthy cells. Conversely, cancer cells do not die. They multiply by division, making 2 cancer cells, then 4, 8, 16, 32 and so on. In lab tests, ellagic acid has shown positive results in causing cancer cells to go through the normal apoptosis process without damaging healthy cells. Chemotherapy, radiation, and most conventional treatments cause the death of cancer cells as well as many healthy cells. This lack of discrimination in the conventional treatments for cancer can possibly destroy the immune system in the process.
US-A-2002/0197341 discloses synergistic mixtures of pomegranate extracts suitable to retard progression of cell proliferation disorders. These mixtures include two or more of pomegranate juice extract, pomegranate pericarp extract and pomegranate seed oil.
Lansky et al. (Investigational New Drugs 23: 121-122, 2005) discloses inhibition of prostate cancer cell invasion by pomegranate pure chemicals ellagic acid, caffeic acid, luteolin and punicic acid.

Accordingly, it is to be appreciated that there is a constant need for compositions capable of preventing disease and promoting health and repair of damaged tissues. The present invention addresses this need.

### SUMMARY OF THE INVENTION

It is well understood that ellagic acid can be found in different foods. However, the bio-availability of the ellagic acid (or ellagitannins) from various plant sources is not well established. It is believed that pomegranate, and particularly, pomegranate seeds, offers an advantageous source of bio-available ellagic acid for use as a food supplement.

The present invention offers a method of extracting ellagic acid (or ellagitannins) from pomegranate seed, and for human oral administration as a nutritional supplement, therapeutic or prophylactic agent.

Accordingly, the present invention teaches a method of providing a food supplement including ellagic acid (or a polyphenol extract, or ellagitannin), and for using this food supplement for beneficial human consumption.

Specifically, the present invention teaches an ellagic acid (ellagitannins) containing, pomegranate-derived, dry powder material for human oral consumption as a food supplement, therapeutic or prophylactic agent, said dry powder material comprising an extract from pomegranate plant materials including pomegranate seed containing from 40% to 90% of ellagic acid (ellagitannins), wherein said dry powder material is obtainable by a method comprising the steps of:
providing a starting material derived from the pomegranate plant;
soaking said starting material in a solution including ethanol;
saving a solution of extraction;
concentrating the solution of extraction to produce a first mother liquid;
extracting the first mother liquid using acetic ether to produce a second solution of extraction;
concentrating the second solution of extraction to produce a second mother liquid;
concentrating the second mother liquid to produce a solid product material; and
reducing said solid product material to a dry powder product material.

The present invention also teaches a method of providing said ellagic acid containing dry powder material, said method comprising the steps of:
providing a starting material derived from the pomegranate plant;
soaking said starting material in a solution including ethanol;
saving a solution of extraction;
concentrating the solution of extraction to produce a first mother liquid;
extracting the first mother liquid using acetic ether to produce a second solution of extraction;
concentrating the second solution of extraction to produce a second.mother liquid;
concentrating the second mother liquid to produce a solid product material; and
reducing said solid product material to a dry powder product material.

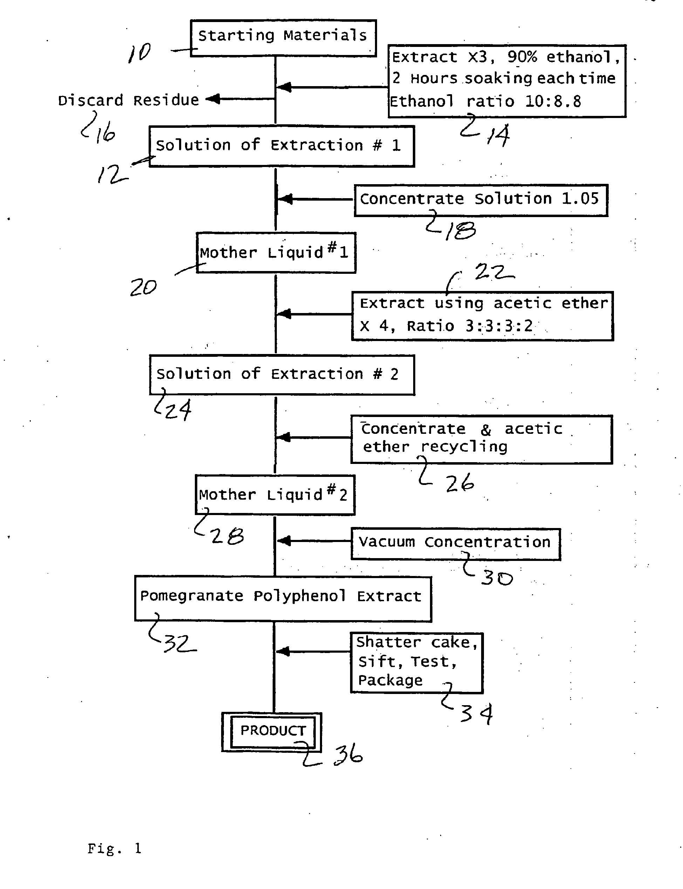

### BRIEF DESCRIPTION OF THE DRAWING

The single drawing Figure is a schematic diagram of a process (series of method steps) for preparing ellagic acid powder for use as a food supplement.

### DETAILED DESCRIPTION OF THE PREFERRED EMBODIMENTS

Considering now the drawing Figure, it is seen that a method of providing ellagic acid as a food supplement according to this invention begins with a stating material, indicated at step 10. Most preferably, the starting material 10 includes or consists essentially of ground or crushed pomegranate seed. As an alternative, the husk or meat of the pomegranate fruit may be included in the starting material. Still alternatively, the starting material may include pomegranate leaves.

In order to produce a first solution of extraction 12, from the starting materials 10, these starting materials are soaked (indicated as step 14 on the Figure) a minimum of 2 hours in an ethanol solution, which may comprise as much as about 90% ethanol. As is noted on the drawing Figure, the solution used to conduct the soaking steps may alternatively be in a ratio of about 10:8.8, which is about 55% ethanol, although the invention is not limited to this precise ratio. The soaking step is preferably repeated three times, with the solution being drained off and saved (possibly accompanied by mild pressing of the residue to remove excess moisture) at the conclusion of each soaking interval. The saved solutions (i.e., from the three soaking steps) are combined and are indicated as solution of extraction #1 on the Figure. As is indicated at 16 on the Figure, the residue (i.e., solid residue) is discarded.

Next, the solution of extraction #1 (i.e., indicated at 12 on the Figure) is concentrated by a ratio of about 1:05 (i.e., about a 20:1 reduction in volume) (indicated at 18 on the Figure) to produce a so-called mother liquid #1 (indicated at 20 on the Figure). This mother liquid #1 is subjected to an extraction (indicated at 22 on the Figure) utilizing acetic ether. The extraction is repeated four (4) times, using a ratio of 3:3:3:2. Consequently, a solution of extraction #2 (indicated at 24 on the Figure) is produced. This solution of extraction #2 is concentrated and acetic ether is recycled (indicated at step 26 on the Figure).

The steps recited above result in a so-called mother liquid #2 (indicated at 28 on the Figure). This mother liquid #2 is next subjected to vacuum concentration (or vacuum drying), indicated at 30 on the Figure, to produce an intermediate product 32. This intermediate product 32 is pomegranate polyphenol extract in a solid, dry cake form. The cake form of intermediate product 32 is most preferably subjected to cake shattering (i.e., breaking, milling, and/or possibly grinding) of the cake form intermediate product, to form a powder. The powder is sifted for size, is tested, and is then packaged (all indicated at 34 on the Figure), to produce the product 36. It is to be noted that dependent upon the particulars of the starting materials 10, and of the process used according to the Figure and the description above, the ellagic acid present in the product 36 may range from about 40% to about 90%.

Most desirably, the dry product 36 is packaged into soft gelatin capsules for oral consumption by humans. That is, the capsules may contain, for example, about 200 mg of ellagic acid in a bio-available form, which may be consumed by humans as a food supplement. This food supplement may have, when orally administrated as a nutritional supplement, therapeutic or prophylactic effects. Further, formulations for oral use may also be presented as hard gelatin capsules wherein the active ingredient is mixed with an inert solid diluent, for example calcium carbonate, calcium phosphate or kaolin, or as soft gelatin capsules wherein the active ingredient is mixed with water or an oil medium, such as peanut oil, liquid paraffin or olive oil.

Further, aqueous or other liquid suspensions may contain the ellagic acid of the invention in admixture with excipients suitable for the manufacture of aqueous suspensions. Such excipients include suspending agents, dispersing or wetting agents, one or more preservatives, one or more coloring agents, one or more flavoring agents and one or more sweetening agents such as sucrose or saccharin. Alternatively, oil suspensions may be formulated by suspending the active ingredient in a vegetable oil, such as arachis oil, olive oil, sesame oil or coconut oil, or in a mineral oil such as liquid paraffin. The oil suspension may contain a thickening agent, such as beeswax, hard paraffin or cetyl alcohol. Sweetening agents, such as those set forth above, and flavoring agents may be added to provide a palatable oral preparation. These compositions may be preserved by an added antioxidant such as ascorbic acid. So too, syrups and elixirs may be formulated with sweetening agents, such as glycerol, sorbitol or sucrose. Such formulations may also contain a demulcent, a preservative, a flavoring agent and/or a coloring agent.

For use as a nutritional supplement, prophylactic or therapeutic agent, the ellagic acid product 36, preferably packaged and presented as explained above, is orally administered in a daily dosage of between about 500 mg and 5,000 mg. More preferably, it is administered in a daily dosage of between about 2,000 mg and 4,000 mg. Most preferably, it is administered in a daily dosage of between about 2,000 and 3,000 mg per day.

The above detailed description of the invention is set forth solely to assist in understanding the invention. It is to be understood that variations of the invention, including all equivalents now known or later developed, are to be considered as falling within the scope of the invention, which is limited only by the following claims.

## Claims

1. An ellagic acid or ellagitannins containing, pomegranate-derived, dry powder material for human oral consumption as a food supplement, therapeutic or prophylactic agent, said dry powder material comprising an extract from pomegranate plant materials including pomegranate seed containing from 40% to 90% of ellagic acid or ellagitannins, wherein said dry powder material is obtainable by a method comprising the steps of:
providing a starting material derived from the pomegranate plant;
soaking said starting material in a solution including ethanol;
saving a solution of extraction;
concentrating the solution of extraction to produce a first mother liquid;
extracting the first mother liquid using acetic ether to produce a second solution of extraction;
concentrating the second solution of extraction to produce a second mother liquid;
concentrating the second mother liquid to produce a solid product material; and
reducing said solid product material to a dry powder product material.

2. The material of Claim 1 wherein said pomegranate plant materials include substantially only pomegranate seed.

3. The material of Claim 1 wherein said dry powder extract is presented as a dry powder packaged in gelatin capsules for human oral consumption.

4. The material of Claim 3 for oral consumption in a daily dosage of from 500 mg to 5,000 mg, more preferably of from 2,000 mg to 4,000 mg, and most preferably of from 2,000 to 3,000 mg.

5. A method of providing an ellagic acid or ellagitannins containing dry powder material for human consumption as defined in Claim 1, said method comprising steps of:
providing a starting material derived from the pomegranate plant;
soaking said starting material in a solution including ethanol;
saving a solution of extraction;
concentrating the solution of extraction to produce a first mother liquid;
extracting the first mother liquid using acetic ether to produce a second solution of extraction;
concentrating the second solution of extraction to produce a second mother liquid; and
concentrating the second mother liquid to produce a solid product material; and
reducing said solid product material to a dry powder product material.

6. The method of Claim 5 wherein said starting material consists essentially of pomegranate seed.

7. The method of Claim 5 wherein said soaking step is repeated plural times, and each time the liquid constituent including ethanol is saved and combined to provide said first solution of extraction.

8. The method of Claim 7 wherein said soaking step is repeated three times.

9. The method of Claim 5 wherein said soaking step uses an ethanol solution of from 50% ethanol to 90% ethanol.

10. The method of Claim 5 wherein said concentrating step to produce said first mother liquid from said first solution of extraction utilizes a concentration of 20 to 1 on a volume basis.

11. The method of Claim 5 wherein said first mother liquid is extracted plural times using acetic ether in order to provide said second solution of extraction.

12. The method of Claim 11 wherein said plural extractions of said first mother liquid to provide said second solution of extraction is performed four times, and the four extractions respectively utilize a 3:3:3:2 ratio of mother liquid to acetic ether.

13. The method of Claim 5 wherein said step of concentrating said second solution of extraction to provide said second mother liquid also includes recycling of said acetic ether.

14. The method of Claim 5 wherein said step of concentrating said second mother liquid to provide said solid product material is performed using vacuum.

15. The method of Claim 5 further including the steps of fracturing said solid product material to produce a dry powder form of product material.

16. A method of providing an ellagic acid or ellagitannins containing food supplement dry powder material according to Claim 5, wherein:
said starting material is derived essentially from the seed of the pomegranate plant;
said starting material is soaked plural times in a solution including ethanol;
the liquid fractions from each soaking step are saved and combined to provide a solution of extraction, and the solid fraction is disposed;
said solution of extraction is concentrated by a ratio of 20:1 to produce a first mother liquid;
said first mother liquid is extracted plural times using acetic ether each time producing a respective extracted solution, and said respective extracted solutions are saved and combined to produce a second solution of extraction;
said second solution of extraction is concentrated to produce a second mother liquid while providing recycling of acetic ether;
said second mother liquid is concentrated to produce a solid product material; and
said solid product material is reduced to a dry powder product material.

17. The method of Claim 16 wherein said starting material consists essentially only of pomegranate seed.

18. The method of Claim 16 wherein said soaking step is repeated three times, and an ethanol solution of 50% ethanol to 90% ethanol is utilized for each soaking operation.

## Patentansprüche

1. Ellagsäure oder Ellagitannine enthaltendes Trockenpulvermaterial aus Granatapfel als Nahrungsergänzungsmittel, Therapeutikum oder Prophylaktikum zur humanen oralen Aufnahme, wobei das Trockenpulvermaterial einen Extrakt aus Granatapfelpflanzenmaterialien einschließlich Granatapfelsamen umfasst, der 40% bis 90% Ellagsäure oder Ellagitannine enthält, wobei das Trockenpulvermaterial mittels eines Verfahrens erhältlich ist, das die Schritte umfasst:
Bereitstellen eines Ausgangsmaterials aus der Granatapfelpflanze;
Weichen lassen des Ausgangsmaterials in einer Lösung, die Ethanol enthält;
Gewinnen einer Extraktionslösung;
Einengen der Extraktionslösung, um eine erste Mutterlauge herzustellen;
Extrahieren der ersten Mutterlauge mit Ethylacetat, um eine zweite Extraktionslösung herzustellen;
Einengen der zweiten Extraktionslösung, um eine zweite Mutterlauge herzustellen;
Einengen der zweiten Mutterlauge, um ein festes Produktmaterial herzustellen; und
Zerkleinern des festen Produktmaterials zu einem Trockenpulverproduktmaterial.

2. Material nach Anspruch 1, wobei die Granatapfelpflanzenmaterialien im Wesentlichen ausschließlich Granatapfelsamen beinhalten.

3. Material nach Anspruch 1, wobei der Trockenpulverextrakt als ein in Gelatinekapseln verpacktes Trockenpulver zur humanen oralen Aufnahme vorliegt.

4. Material nach Anspruch 3 zur oralen Aufnahme in einer Tagesdosis von 500 mg bis 5.000 mg, bevorzugt von 2.000 mg bis 4.000 mg und besonders bevorzugt von 2.000 bis 3.000 mg.

5. Verfahren zum Bereitstellen eines Ellagsäure oder Ellagitannine enthaltenden Trockenpulvermaterials zur humanen Aufnahme nach Anspruch 1, wobei das Verfahren die Schritte umfasst:
Bereitstellen eines Ausgangsmaterials aus der Granatapfelpflanze;
Weichen lassen des Ausgangsmaterials in einer Lösung, die Ethanol enthält;
Gewinnen einer Extraktionslösung;
Einengen der Extraktionslösung, um eine erste Mutterlauge herzustellen;
Extrahieren der ersten Mutterlauge mit Ethylacetat, um eine zweite Extraktionslösung herzustellen;
Einengen der zweiten Extraktionslösung, um eine zweite Mutterlauge herzustellen;
Einengen der zweiten Mutterlauge, um ein festes Produktmaterial herzustellen; und
Zerkleinern des festen Produktmaterials zu einem Trockenpulverproduktmaterial.

6. Verfahren nach Anspruch 5, wobei das Ausgangsmaterial im Wesentlichen aus Granatapfelsamen besteht.

7. Verfahren nach Anspruch 5, wobei der Weichschritt mehrmals wiederholt wird, und die flüssige Komponente, die Ethanol enthält, jedes Mal gewonnen und zur ersten Extraktionslösung vereinigt wird.

8. Verfahren nach Anspruch 7, wobei der Weichschritt dreimal wiederholt wird.

9. Verfahren nach Anspruch 5, wobei bei dem Weichschritt eine Ethanollösung mit 50% Ethanol bis 90% Ethanol verwendet wird.

10. Verfahren nach Anspruch 5, wobei bei dem Schritt des Einengens zur Herstellung der ersten Mutterlauge aus der ersten Extraktionslösung ein Einengen im Volumen von 20 zu 1 erfolgt.

11. Verfahren nach Anspruch 5, wobei die erste Mutterlauge zum Bereitstellen der zweiten Extraktionslösung mehrmals mit Ethylacetat extrahiert wird.

12. Verfahren nach Anspruch 11, wobei die mehrmalige Extraktion der ersten Mutterlauge zum Bereitstellen der zweiten Extraktionslösung viermal erfolgt und bei den vier Extraktionen respektive ein 3:3:3:2-Verhältnis von Mutterlauge zu Ethylacetat verwendet wird.

13. Verfahren nach Anspruch 5, wobei der Schritt des Einengens der zweiten Extraktionslösung zum Bereitstellen der zweiten Mutterlauge Rück führung des Ethylacetats beinhaltet.

14. Verfahren nach Anspruch 5, wobei der Schritt des Einengens der zweiten Mutterlauge zum Bereitstellen des festen Produktmaterials unter Vakuum erfolgt.

15. Verfahren nach Anspruch 5, weiterhin umfassend die Schritte des Zerbrechens des festen Produktmaterials zur Bildung eines Produktmaterials in Trockenpulverform.

16. Verfahren zum Bereitstellen eines Ellagsäure oder Ellagitannine enthaltenden Nahrungsergänzungsmittel-Trockenpulvermaterials gemäß Anspruch 5, wobei:
das Ausgangsmaterial im Wesentlichen aus dem Samen der Granatapfelpflanze stammt;
das Ausgangsmaterial mehrmals in einer Lösung, die Ethanol enthält, geweicht wird;
die Flüssigfraktionen eines jeden Weichschritts gewonnen und zu einer Extraktionslösung vereinigt werden und die feste Fraktion verworfen wird;
die Extraktionslösung zur Herstellung einer ersten Mutterlauge in einem Verhältnis von 20:1 eingeengt wird;
die erste Mutterlauge mehrmals mit Ethylacetat extrahiert wird, wobei jedes Mal eine extrahierte Lösung hergestellt wird, und die extrahierten Lösungen gewonnen und zu einer zweiten Extraktionslösung vereinigt werden;
die zweite Extraktionslösung unter Rückführung von Ethylacetat zu einer zweiten Mutterlauge eingeengt wird;
die zweite Mutterlauge zu einem festen Produktmaterial eingeengt wird; und
das feste Produktmaterial zu einem Trockenpulverproduktmaterial zerkleinert wird.

17. Verfahren nach Anspruch 16, wobei das Ausgangsmaterial im Wesentlichen ausschließlich aus Granatapfelsamen besteht.

18. Verfahren nach Anspruch 16, wobei der Weichschritt dreimal wiederholt wird und für jeden Weichvorgang eine Ethanollösung mit 50% Ethanol bis 90% Ethanol verwendet wird.

## Revendications

1. Matériau en poudre sèche dérivé de grenade contenant de l'acide ellagique ou de l'ellagitannine, pour la consommation orale humaine comme complément alimentaire, agent thérapeutique ou prophylactique, ledit matériau en poudre sèche comprenant un extrait de matériaux à base de grenade comprenant les graines de grenade contenant de 40 % à 90 % d'acide ellagique (ellagitannine), ledit matériau en poudre sèche pouvant être obtenu par un procédé comprenant les étapes consistant à :
se munir d'un matériau de départ dérivé de la grenade ;
faire tremper le matériau de départ dans une solution comprenant de l'éthanol ;
conserver une solution d'extraction ;
concentrer la solution d'extraction pour produire une première liqueur mère ;
extraire la première liqueur mère en utilisant de l'éther acétique pour produire une deuxième solution d'extraction ;
concentrer la seconde solution d'extraction pour produire une seconde liqueur mère ;
concentrer la seconde liqueur mère pour produire un matériau de produit solide ; et
réduire ledit matériau de produit solide en un matériau de produit en poudre sèche.

2. Matériau selon la revendication 1, dans lequel lesdits matériaux à base de grenade comprennent substantiellement, uniquement des graines de grenade.

3. Matériau selon la revendication 1, dans lequel ledit extrait en poudre sèche est présenté en tant que poudre sèche empaquetée dans des capsules de gélatine pour la consommation orale humaine.

4. Matériau selon la revendication 3 pour la consommation orale en une posologie quotidienne de 500 mg à 5 000 mg, de préférence de 2 000 mg à 4 000 mg et de manière préférée entre toutes, de 2 000 mg à 3 000 mg.

5. Procédé permettant d'obtenir un matériau en poudre sèche contenant de l'acide ellagique ou de l'ellagitannine pour la consommation humaine selon la revendication 1, ledit procédé comprenant les étapes consistant à :
se munir d'un matériau de départ dérivé de la grenade ;
faire tremper le matériau de départ dans une solution comprenant de l'éthanol ;
conserver une solution d'extraction ;
concentrer la solution d'extraction pour produire une première liqueur mère ;
extraire la première liqueur mère en utilisant de l'éther acétique pour produire une deuxième solution d'extraction ;
concentrer la seconde solution d'extraction pour produire une seconde liqueur mère ; et
concentrer la seconde liqueur mère pour produire un matériau de produit solide ; et
réduire ledit matériau de produit solide en un matériau de produit en poudre sèche.

6. Procédé selon la revendication 5, dans lequel ledit matériau de départ consiste essentiellement en graines de grenade.

7. Procédé selon la revendication 5, dans lequel l'étape de trempage est répétée à plusieurs reprises et chaque fois, le constituant liquide comprenant de l'éthanol est conservé et combiné pour donner ladite première solution d'extraction.

8. Procédé selon la revendication 7, dans lequel ladite étape de trempage est répétée trois fois.

9. Procédé selon la revendication 5, dans lequel ladite étape de trempage utilise une solution d'éthanol de 50 % d'éthanol à 90 % d'éthanol.

10. Procédé selon la revendication 5, dans lequel ladite étape de concentration pour produire ladite première liqueur mère de ladite première solution d'extraction utilise une concentration de 20 à 1 sur une base volumique.

11. Procédé selon la revendication 5, dans lequel ladite première liqueur mère est extraite plusieurs fois en utilisant de l'éther acétique pour donner ladite seconde solution d'extraction.

12. Procédé selon la revendication 11, dans lequel lesdites multiples extractions de ladite première liqueur mère pour donner ladite seconde solution d'extraction sont réalisées quatre fois et les quatre extractions utilisent respectivement un rapport de 3:3:3:2 de la liqueur mère à l'éther acétique.

13. Procédé selon la revendication 5, dans lequel ladite étape de concentration de ladite seconde solution d'extraction pour donner ladite seconde liqueur mère comprend également le recyclage dudit éther acétique.

14. Procédé selon la revendication 5, dans lequel ladite étape de concentration de ladite seconde liqueur mère pour donner ledit matériau de produit solide est réalisé sous vide.

15. Procédé selon la revendication 5, comprenant en outre les étapes consistant à fracturer ledit matériau de produit solide pour produire une forme de poudre sèche du matériau du produit.

16. Procédé permettant d'obtenir un matériau en poudre sèche en tant que complément alimentaire contenant de l'acide ellagique ou de l'ellagitannine selon la revendication 5, dans lequel :
ledit matériau de départ est dérivé essentiellement des graines de la grenade ;
ledit matériau de départ est trempé plusieurs fois dans une solution comprenant de l'éthanol ;
les fractions liquides de chaque étape de trempage sont conservées et combinées pour donner une solution d'extraction et la fraction solide est éliminée ;
ladite solution d'extraction est concentrée en un rapport de 20:1 pour produire une première liqueur mère ;
ladite première liqueur mère est extraite plusieurs fois en utilisant chaque fois de l'éther acétique pour produire une solution extraite respective et lesdites solutions extraites respectives sont conservées et combinées pour produire une seconde solution d'extraction ;
ladite seconde solution d'extraction est concentrée pour produire une seconde liqueur mère tout en assurant le recyclage de l'éther acétique ;
ladite seconde liqueur mère est concentrée pour produire un matériau de produit solide ; et
ledit matériau de produit solide est réduit en un matériau de produit en poudre sèche.

17. Procédé selon la revendication 16, dans lequel ledit matériau de départ consiste essentiellement uniquement en graines de grenade.

18. Procédé selon la revendication 16, dans lequel ladite étape de trempage est répétée trois fois et une solution d'éthanol de 50 % d'éthanol à 90 % d'éthanol est utilisée pour chaque opération de trempage.
